# EUROPEAN PATENT SPECIFICATION

(11) **EP 0 410 361 B1**
(45) Date of publication and mention of the grant of the patent: **28.02.1996**
(21) Application number: 90114117.6
(22) Date of filing: 24.07.1990
(51) Int. Cl.: C08F 12/04

(54) **Process for producing styrene polymers**
Verfahren zur Herstellung von Styrolpolymeren
Procédé de préparation de polymères de styrènes

(30) Priority: 28.07.1989 JP 193878/89; 28.07.1989 JP 193879/89
(43) Date of publication of application: 30.01.1991
(60) Divisional application: 95101613.8
(73) Proprietor: IDEMITSU KOSAN COMPANY LIMITED, Tokyo 100 (JP)
(72) Inventor: Teshima, Hideo, Ichihara-shi, Chiba-ken (JP); Kuramoto, Masahiko, Ichihara-shi, Chiba-ken (JP); Takeuchi, Mizutomo, Ichihara-shi, Chiba-ken (JP)
(74) Representative: Türk, Gille, Hrabal, Leifert

(56) References cited:
- EP-A- 0 091 101
- EP-A- 0 224 097
- FR-A- 1 361 512
- US-A- 2 210 639

## Description

### BACKGROUND OF THE INVENTION

### 1. Field of the Invention

This invention relates to a process for producing styrene polymers, more specifically to a process for producing styrene polymers having a high degree of syndiotactic configuration in their stereochemical structure of the polymer chain with good efficiency by using a purified styrene monomer as a starting material.

### 2. Description of the Related Arts

Heretofore, styrene polymers produced by the radical polymerization method have an atactic configuration in stereostructure and are molded to various shapes by various molding methods such as injection molding, extrusion molding, blow molding, vacuum molding and cast molding, and they have been widely used as domestic electric appliances, office machines, household goods, packaging containers, toys, furnitures, synthetic papers and other industrial materials.

However, such styrene polymers having atactic configuration have disadvantage that it is inferior in heat resistance and chemical resistance.

The group of the present inventors has previously succeeded in developing styrene polymers having a high degree of syndiotacticity and further proposed that styrene polymers having syndiotactic configuration by using a catalyst comprising a transition metal compound and a reaction product (aluminoxiane) of an organoaluminum compound with a condensing agent (see Japanese Patent Application Laid-Open No. 187708/1987, etc.).

However, this polymerization catalyst is poisoned by impurities in the reaction system whereby activity thereof is likely lowered and thus it would be desired to use a highly purified styrene monomer as a starting material.

In the course of the research, it has been found that a phenylacetylene compound contained in a styrene monomer for industrial use particularly poisons polymerization catalyst markedly. For example, commercially available styrene monomers for industrial use contain impurities such as phenylacetylene in an amount of 80 to 300 ppm and these impurities cause poisoning of catalyst whereby catalyst activity is markedly lowered. Particularly, phenylacetylene has boiling point near that of a styrene monomer so that separation by distillation would be difficult.

A method for purifying styrene monomer in which a monomer is treated by silica gel or activated charcoal has heretofore been known (Japanese Patent Publication No. 38543/1977), but the method should be utilized only for production of atactic polystyrenes. Thus, the purification procedure of the styrene monomer has mainly carried out to remove a specific organic polymerization inhibitor contained in the styrene monomer as an impurity.

Also, in Japanese Patent Application Laid-Open No. 4433/1973, there is described a method in which a crude styrene is treated with iron-substituted zeolite for purifying. However, zeolite type absorbents have a problem that it causes polymerization reaction generating polystyrene having atactic configuration during purification procedure.

Thus, the present inventors have intensively studied to develop a process for removing impurities which poison catalysts to be used in the process for producing styrene polymers having syndiotactic configuration from a styrene monomer used as a starting material with moderate conditions.

### SUMMARY OF THE INVENTION

Under such a situation, the present inventors have conducted various attempts. As the results, it has been found that a styrene monomer containing phenylacetylene compounds in an amount of 50 ppm or less which can be obtained by subjecting commercially available styrene monomers for industry to various purification processings could markedly reduce poisoning of a polymerization catalyst. It has also been found that, when such a purified styrene monomer is employed, styrene polymers having syndiotactic configuration could be obtained markedly effectively. The present invention has been accomplished based on such findings.

That is, the present invention relates to a process for producing styrene polymers having a high degree of syndiotactic configuration by polymerizing a styrene monomer in the presence of a catalyst composed of (A) aluminoxane and (B) a transition metal compound, selected from the group of zirconium, hafnium, titanium and vanadium compounds, characterized in that a purified styrene monomer containing phenylacetylene compounds in an amount of 50 ppm or less is used as a starting material.

### PREFERRED EMBODIMENTS OF THE INVENTION

The styrene monomer to be used in the process of the present invention may include various ones but usually those represented by the following formula (I): 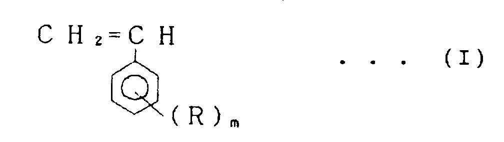 wherein R represents a hydrogen atom, a halogen atom or a hydrocarbon group having carbon atoms of 20 or less; and m is an integer of 1 to 3, provided that, when m is plural number, each R may be the same or different.

In the above formula, each symbol is as mentioned above, but more specifically, R represents a hydrogen atom, a halogen atom (e.g. chlorine, bromine, fluorine, iodine) or a hydrocarbon group having carbon atoms of 20 or less, preferably 10 to 1 carbon atom (e.g. a saturated hydrocarbon group (particularly an alkyl group) such as a methyl group, an ethyl group, a propyl group, a butyl group, a pentyl group and a hexyl group; or an unsaturated hydrocarbon group such as a vinyl group). Examples of the styrene monomer represented by the formula (I) may include alkylstyrenes such as styrene, p-methylstyrene, m-methylstyrene, o-methylstyrene, 2,4-dimethylstyrene, 2,5-dimethylstyrene, 3,4-dimethylstyrene, 3,5-dimethylstyrene, p-ethylstyrene, m-ethylstyrene and p-tertiary-butylstyrene; p-divinylbenzene; m-divinylbenzene; trivinylbenzene; halogenated styrenes such as p-chlorostyrene, m-chlorostyrene, o-chlorostyrene, p-bromostyrene, m-bromostyrene, o-bromostyrene, p-fluorostyrene, m-fluorostyrene, o-fluorostyrene and o-methyl-p-fluorostyrene, or two or more of mixtures thereof.

In styrene monomers commercially available or provided as a raw material for industry, in addition to t-butylcatechol which is a polymerization inhibitor, water, corresponding aldehyde (benzaldehyde in the case of styrene), corresponding acetylene (phenylacetylene in the case of styrene), corresponding peralkylating material (α-methylstyrene or β-methylstyrene in the case of styrene) are contained. Particularly, phenylacetylene in the styrene has boiling point near that of styrene so that separation of phenylacetylene from styrene is difficult by the usual distillation.

In the present invention, prior to polymerizing a styrene monomer containing impurities used as starting materials in industry, it is necessary to carry out the purification processing to reduce the content of phenylacetylene compound in said monomers to 50 ppm or less, preferable 10 ppm or less. If the amount of the phenylacetylene compound in the styrene monomer exceeds 50 ppm, poison of a polymerization catalyst is remarkable so that polymerization can hardly proceed with good efficiency.

The phenylacetylene compound may include various ones but it is usually represented by the following formula (II): 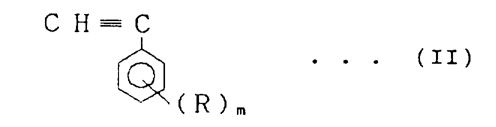 wherein R represents a hydrogen atom, a halogen atom or a hydrocarbon group having carbon atoms of 20 or less; and m is an integer of 1 to 3, provided that, when m is plural number, each R may be the same or different.

In the above formula, each symbol is as mentioned above, but more specifically, R represents a hydrogen atom, a halogen atom (e.g. chlorine, bromine, fluorine, iodine) or a hydrocarbon group having carbon atoms of 20 or less, preferably 10 to 1 carbon atom (e.g. a saturated hydrocarbon group (particularly an alkyl group) such as a methyl group, an ethyl group, a propyl group, a butyl group, a pentyl group and a hexyl group; or an unsaturated hydrocarbon group such as a vinyl group). Examples of the phenylacetylene represented by the formula (II) may include alkylphenylacetylenes such as phenylacetylene, p-methylphenylacetylene, m-methylphenylacetylene, o-methylphenylacetylene, 2,4-dimethylphenylacetylene, 2,5-dimethylphenylacetylene, 3,4-dimethylphenylacetylene, 3,5-dimethylphenylacetylene, p-ethylphenylacetylene, m-ethylphenylacetylene and p-tertiary-butylphenylacetylene; p-divinylbenzene; m-divinylbenzene; trivinylbenzene; halogenated phenylacetylenes such as p-chlorophenylacetylene, m-chlorophenylacetylene, o-chlorophenylacetylene, p-bromophenylacetylene, m-bromophenylacetylene, o-bromophenylacetylene, p-fluorophenylacetylene, m-fluorophenylacetylene, o-fluorophenylacetylene and o-methyl-p-fluorophenylacetylene, or two or more of mixtures thereof.

Here, there are various methods for purifying the styrene monomer and the methods are not particularly limited so long as they can reduce the content of phenylacetylene compound in the monomer to 50 ppm or less. As the concrete method, the following purification methods I to VII are mentioned.
(1) A method in which a styrene monomer is treated with at least one inorganic substance selected from the group consisting of alumina, activated alumina, silica gel, activated charcoal, calcium oxide, aluminum silicate and diatomaceous earth (Purification method I).
(2) A method in which a styrene monomer is degassed under reduced pressure or passed a dried inert gas by bubbling therethrough to remove water therein and then treated with the above inorganic substance (Purification method II).
(3) A method in which a styrene monomer is contacted with an organoaluminum and then treated with the above inorganic substance (Purification method III).
(4) A method in which a styrene monomer is contacted with an organoaluminum and then distilled (Purification method IV).
(5) A method in which a styrene monomer is contacted with an organoaluminum and then subjected to hydrolysis and distilled (Purification method V).
(6) A method in which a styrene monomer is contacted with an organoaluminum and then subjected to hydrolysis and treated with the above inorganic substance (Purification method VI).
(7) A method in which a styrene monomer is subjected to hydrogenation treatment in the presence of a hydrogenation catalyst to selectively hydrogenate phenylacetylene compound contained therein (Purification method VII).

Firstly, according to Purification method I, prior to effecting polymerization of a styrene monomer as a raw material for industry which contain impurities, such a monomer is treated with an inorganic substance such as alumina, activated alumina, silica gel, activated charcoal, calcium oxide, aluminum silicate and diatomaceous earth, particularly preferably with alumina or activated alumina, whereby poisoning of a polymerization catalyst can be decreased. The treatment by alumina, etc. at this time can be carried out under various conditions but usually it can be carried out by contacting the above materials with a styrene monomer at a temperature of 0 to 50°C for 10 minutes to 2 hours. Incidentally, zeolites are not suitable as an inorganic material for the above treatment because they cause polymerization reaction in the course of the treatment to form styrene polymers having atactic configuration.

Also, according to Purification method II, it is effective to remove particularly water in the styrene monomer by degassing the starting styrene monomer under reduced pressure or passing a dried inert gas through the starting styrene monomer by bubbling prior to the processing of the above Purification method I. A standard of removal at this case may be optionally selected depending on the situation, and usually water is removed so as to become the water content to 5 ppm or less by degassing under reduced pressure or passing dried gas therethrough by means of bubbling.

Next, according to Purification methods III to VI, the above styrene monomer is firstly brought into contact with an organoaluminum compound. Here, the organoaluminum compound includes a compound represented by the formula:

AlR¹ ᵢR ⱼR³ ₃₋₍ᵢ₊ⱼ₎ (III)

wherein R¹, R and R³ each represent a hydrogen atom, an alkyl group or an alkoxy group having 1 to 8 carbon atoms, or a halogen atom; i and j each are integers of 0 to 3 and i + j is an integer of 0 to 3.

Specific examples of the organoaluminum compound include trimethylaluminum, triethylaluminum, tripropylaluminum, triisobutylaluminum, trioctylaluminum, dimethylaluminum chloride, diethylaluminum chloride, hydrogenated diethylaluminum, hydrogenated diisobutylaluminum and diethylaluminum ethoxide. Of these compounds, an organoaluminum compound containing no halogen atom is preferred, and an organoaluminum compound having hydrogen, an ethyl group and/or isobutyl group such as triisobutylaluminum, hydrogenated diisobutylaluminum and hydrogenated diethylaluminum is more preferred. Also, the aluminoxane of the component (A) as the catalyst may be used.

Prior to contact with the above organoaluminum compound, it is preferred to measure amounts of impurities such as phenylacetylene compound in the styrene monomer by a gas chromatography or the like. Based on the measurement, the organoaluminum compound which is to be brought into contact with monomers is optionally added within the range of 1 to 1000 moles per mole of impurity, but in many cases, it is sufficient within the range of 1 to 10 moles. However, even when excessive amount of the organoaluminum compound is added to the reaction system, no particular problem would be caused. The reaction temperature is 0 to 100°C, preferably 20 to 40°C.

The reaction time is 5 minutes to 10 hours, but in many cases, it is sufficient with the reaction time of 10 minutes to 1 hour.

In Purification methods III to VI as mentioned above, the processing after brought into contact with the organoaluminum compound is any of the following. That is, (a) processing with the above inorganic material (Purification method III), (b) direct distillation (Purification method IV), (c) after hydrolysis, distillation (Purification method V) or (d) after hydrolysis, processing with the above inorganic material (Purification method VI).

In case where after brought into contact with the organoaluminum compound, distillation is directly carried out (Purification method IV), distillation procedure may be carried out under the conditions of separating the styrene monomer from the reaction product of the organoaluminum compound and impurities. The distillation conditions can be optionally selected, but distillation at a low temperature is preferred and that under reduced pressure is more preferred than that under atmospheric pressure. The distillation temperature is 0 to 100°C, preferably 0 to 50°C. To the contrary, when distillation is carried out after hydrolysis (Purification method V), distillation can be carried out so as to separate the styrene monomer from a hydrolysis product of a reaction product composed of the organoaluminum compound and impurities in the styrene monomer. Since the organoaluminum compound used is also hydrolyzed to form aluminum hydroxide, it is preferred to distillate after separation of an organic phase comprising the styrene monomer and an aqueous phase containing aluminum hydroxide and the like. Also, when the processing with the above inorganic material is carried out after hydrolysis (Purification method VI), hydrolysis can be carried out in the same operations and conditions as mentioned above and then the hydrolyzed product is removed by adsorbing to the above inorganic material.

Next, according to Purification method VII, by subjecting the above styrene monomer to hydrogenation treatment in the presence of a hydrogenation catalyst containing a platinum group metal (such as Pt, Pd, Rh and Ir) or Ni and Cu, phenylacetylene compounds contained therein can be selectively hydrogenated to be removed.

Of these processings, the above Purification method III is particularly suitable since no distillation processing nor addition of water is accompanied by.

In the process of the present invention, styrene polymers having high syndiotacticity can be obtained with high activity by polymerizing the styrene monomers purified as mentioned above in the presence of a polymerization catalyst.

The styrene polymers produced by the process of the present invention has a high degree of syndiotactic configuration. Here, the styrene polymer which has a high degree of the syndiotactic configuration means that its stereochemical structure is mainly the syndiotactic configuration, i.e. the stereostructure in which phenyl groups or substituted phenyl groups as side chains are located alternately at opposite directions (racemic isomer) relative to the main chain consisting of carbon-carbon bonds. Tacticity is quantitatively determined by the nuclear magnetic resonance method (¹³C-NMR method) using carbon isotope. The tacticity as determined by the ¹³C-NMR method can be indicated in terms of proportions of structural units continuously connected to each other, i.e. a diad in which two structural units are connected to each other, a triad in which three structural units are connected to each other and a pentad in which five structural units are connected to each other. The styrene polymers having such a high degree of syndiotactic configuration means that the proportion of racemic diad is usually at least 75% and preferably at least 85%, or the proportions of racemic pentad is at least 30% and preferably at least 50%. However, the degree of the syndiotactic configuration may somewhat vary depending on the kind of the substituent or the content of each recurring unit.

As the catalyst to be used in the process of the present invention, those comprising (A) aluminoxane and (B) a transition metal compound are suitable.

The aluminoxane which is Component (A) of the catalyst is a compound obtained by contacting with various organoaluminum compound and a condensing agent. As the organoaluminum compound used as a starting material, an organoaluminum compound represented by the general formula:

AlR⁴ ₃ (IV)

wherein R⁴ is an alkyl group having 1 to 8 carbon atoms,
more specifically, trimethylaluminum, triethylaluminum and triisobutylaluminum can be mentioned, and trimethylaluminum is particularly preferred.

On the other hand, a typical example of the condensing agent for said organoaluminum compound is water. In addition, any compounds capable of undergoing a condensation reaction with organoaluminum compounds including alkylaluminum can be used.

As the aluminoxane of Component (A) may include chain alkylaluminoxane represented by the formula: 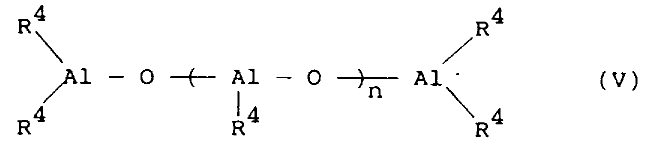
wherein n indicates polymerization degree, and a number of 2 to 50; and R⁴ represents an alkyl group having 1 to 8 carbon atoms,
and cycloalkylaluminoxane having the repeating unit represented by the general formula: 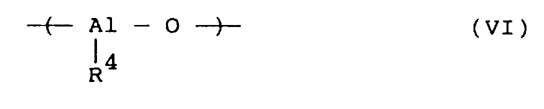 Of these alkylaluminoxanes, that wherein R⁴ is a methyl group, i.e. methylaluminoxane is particularly preferred.

Generally, the reaction product of alkylaluminum compound such as trialkylaluminum and water includes the abovementioned chain alkylaluminoxane and cycloalkylaluminoxane, unreacted trialkylaluminum, a mixture of various condensation products, and further complicatedly associated molecules thereof, which becomes various products according to the contacting conditions of the alkylaluminum compound and water.

The reaction of the alklyaluminum compound and water is not specified, but can be performed according to known methods; for example, (1) a method in which an alkylaluminum compound is dissolved in an organic solvent and then contacted with water; (2) a method in which an alkylaluminum compound is added at the time of polymerization, and then water is added; and (3) a method in which an alkylaluminum compound is reacted with water of crystallization as contained in metal salts, or water absorbed on inorganic or organic compounds. The above water may contain ammonia, amine such as ethylamine, sulfur compound such as hydrogen sulfide, phosphorus compound such as phosphite in the proportion of less than 20%.

The preferred alkylaluminoxane to be used in the present invention is prepared by the method in which, when a hydrated compound is used, the resultant solid residue is filtered after the above contact reaction and the filtrate is heated under atmospheric pressure or reduced pressure at a temperature of 30 to 200°C, preferably 40 to 150°C for from 20 minutes to 8 hours, preferably from 30 minutes to 5 hours while removing the solvent. The temperature for the heat treatment, may be determined optionally depending on various conditions, but usually the above range can be used. If the temperature is less than 30°C, effects cannot be obtained, and if it exceeds 200°C, aluminoxane itself is pyrolyzed. Depending on the conditions of the heat treatment, the reaction product can be obtained as a colorless solid or solution. The product thus obtained can be used as a catalyst solution, if necessary, by dissolving or diluting with a hydrocarbon solvent.

Suitable examples of the alkylaluminoxane are those in which the area of the high magnetic field component in the methyl proton signal region due to the aluminum-methyl group (Al-CH₃) bond as observed by the proton nuclear magnetic resonance method is not more than 50% of the total signal area. That is, in a proton nuclear magnetic resonance (¹H-NMR) spectral analysis of a solution of the alkylaluminoxane in toluene at room temperature, the methyl proton signal due to Al-CH₃ is observed in the region of 1.0 to -0.5 ppm (tetramethylsilane (TMS) standard). Since the proton signal of TMS (0 ppm) is in the 1.0 to -0.5 ppm region of the methyl proton signal due to Al-CH₃, the alkylaluminoxane is measured with toluene as the solvent as the standard. The methyl proton signal due to Al-CH₃ is divided into two components: the high magnetic field component in the -0.1 to -0.5 ppm region and the other magnetic field component in the 1.0 to -0.1 ppm region. In alkylaluminoxane preferably used in the present invention, the area of the high magnetic field component is not more than 50%, preferably 45 to 5% of the total signal area in the 1.0 to -0.5 ppm region.

To the contrary, the transition metal compound of Component (B) to be used as a catalyst of the present invention is a titanium compound, a zirconium compound, a hafnium compound or a vanadium compound. Various titanium compounds can be used and a preferred example is at least one compound selected from the group consisting of titanium compounds and titanium chelate compounds represented by the general formula:

TiR⁵ ₐR⁶ _{b}R⁷ _{c}R⁸ _{4-(a+b+c)} (VII)

or

TiR⁵ _{d}R⁶ ₑR⁷ _{3-(d+e)} (VIII)

wherein R⁵, R⁷ and R⁸ are each a hydrogen atom, an alkyl group having 1 to 20 carbon atoms, an alkoxy group having 1 to 20 carbon atoms, an aryl group having 6 to 20 carbon atoms, an alkylaryl group, an arylalkyl group, an acyloxy group having 1 to 20 carbon atoms, a cyclopentadienyl group, a substituted cyclopentadienyl group, an indenyl group or a halogen atom; a, b and c are each an integer of 0 to 4; and d and e are each an integer of 0 to 3.

R⁵, R⁶, R⁷ and R⁸ in the formulae (VII) and (VIII) each represent a hydrogen atom, an alkyl group having 1 to 20 carbon atoms (specifically, methyl group, ethyl group, propyl group, butyl group, amyl group, isoamyl group, isobutyl group, octyl group and 2-ethylhexyl group), an alkoxy group having 1 to 20 carbon atoms (specifically, methoxy group, ethoxy group, propoxy group, butoxy group, amyloxy group, hexyloxy group, 2-ethylhexyloxy group and phenoxy group), an aryl group having 6 to 20 carbon atoms, an alkylaryl group, an arylalkyl group (specifically, phenyl group, tolyl group, xylyl group and benzyl group), an acyloxy group having 1 to 20 carbon atoms (specifically, heptadecylcarbonyloxy group), a cyclopentadienyl group, a substituted cyclopentadienyl group (specifically, methylcyclopentadienyl group, 1,2-dimethylcyclopentadienyl group and pentamethylcyclopentadienyl group), an indenyl group or a halogen atom (specifically, chlorine, bromine, iodine and fluorine). There R⁵, R⁶, R⁷ and R⁸ may be the same as or different from each other. Furthermore, a, b and c each are an integer of 0 to 4, and d and e each are an integer of 0 to 3.

More preferred titanium compounds include a titanium compound represented by the formula:

TiR''XYZ (IX)

wherein R" represents a cyclopentadienyl group, a substituted cyclopentadienyl group or an indenyl group; X, Y and Z are independently a hydrogen atom, an alkyl group having 1 to 12 carbon atoms, an alkoxy group having 1 to 12 carbon atoms, an aryl group having 6 to 20 carbon atoms, an aryloxy group having 6 to 20 carbon atoms, an arylalkyl group having 6 to 20 carbon atoms or a halogen atom.

The substituted cyclopentadienyl group represented by R" in the above formula is, for example, a cyclopentadienyl group substituted by at least one of an alkyl group having 1 to 6 carbon atoms, more specifically, methylcyclopentadienyl group, 1,2-dimethylcyolopentadienyl group and pentamethylcyclopentadienyl group. In addition, X, Y and Z are each independently a hydrogen atom, an alkyl group having 1 to 12 carbon atoms (specifically, methyl group, ethyl group, propyl group, n-butyl group, isobutyl group, amyl group, isoamyl group, octyl group and 2-ethylhexyl group), an alkoxy group having 1 to 12 carbon atoms (specifically, methoxy group, ethoxy group, propoxy group, butoxy group, amyloxy group, hexyloxy group, octyloxy group and 2-ethylhexyl group), an aryl group having 6 to 20 carbon atoms (specifically, phenyl group and naphthyl group), an aryloxy group having 6 to 20 carbon atoms (specifically, phenoxy group), an arylalkyl group having 6 to 20 carbon atoms (specifically, benzyl group) or a halogen atom (specifically, chlorine, bromine, iodine and fluorine).

Specific examples of the titanium compound represented by the formula (IX) include cyclopentadienyltrimethyltitanium, cyclopentadienyltriethyltitanium, cyclopentadienyltripropyltitanium, cyclopentadienyltributyltitanium, methylcyclopentadienyltrimethyltitanium, 1,2-dimethylcyclopentadienyltrimethyltitanium, 1,3-dimethylcyclopentadienyltrimethyltitanium, pentamethylcyclopentadienyltrimethyltitanium, pentamethylcyclopentadienyltriethyltitanium, pentamethylcyclopentadienyltripropyltitanium, pentamethylcyclopentadienyltributyltitanium, cyclopentadienylmethyltitanium dichloride, cyclopentadienylethyltitanium dichloride, pentamethylcyclopentadienylmethyltitanium dichloride, pentamethylcyclopentadienylethyltitanium dichloride, cyclopentadienyldimethyltitanium monochloride, cyclopentadienyldiethyltitanium monochloride, cyclopentadienyltitanium trimethoxide, cyclopentadienyltitanium triethoxide. cyclopentadienyltitanium tripropoxide, cyclopentadienyltitanium triphenoxide, pentamethylcyclopentadienyltitanium trimethoxide, pentamethylcyclopentadienyltitanium triethoxide, pentamethylcyclopentadienyltitanium tripropoxide, pentamethylcyclopentadienyltitanium tributoxide, pentamethylcyclopentadienyltitanium triphenoxide, cyclopentadienyltitanium trichloride, pentamethylcyclopentadienyltitanium trichloride, cyclopentadienylmethoxyltitanium dichloride, cyclopentadienyldimethoxytitanium chloride, pentamethylcyclopentadienylmethoxytitanium dichloride, cyclopentadienyltribenzyltitanium, pentamethylcyclopentadienylmethyldiethoxytitanium. indenyltitanium trichloride, indenyltitanium trimethoxide, indenyltitanium triethoxide, indenyltrimethyltitanium and indenyltribenzyltitanium.

Of these titanium compounds, a compound containing no halogen atom is preferred and a titanium compound having one π electron type ligand as mentioned above is particularly preferred.

Furthermore, a condensed titanium compound represented by the following formula (X) can be used as the titanium compound. 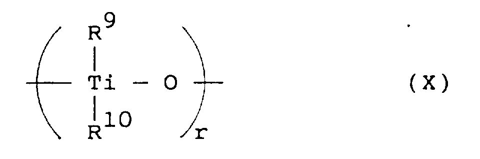 wherein R⁹ and R¹⁰ each represent a halogen atom, an alkoxy group having 1 to 20 carbon atoms or an acyloxy group; and r is an integer of 2 to 20.

Furthermore, the above titanium compounds can be used in the form of a complex formed With an ester or an ether.

The trivalent titanium compound represented by the formula (VIII) typically includes a trihalogenated titanium such as titanium trichloride; and a cyclopentadienyltitanium compound such as cyclopentadienyltitanium dichloride, and also those obtained by reducing a tetravalent titanium compound. These trivalent titanium compounds can be used in the form of a complex formed with an ester or an ether.

In addition, the zirconium compound used as the transition metal compound includes tetrabenzylzirconium, zirconium tetraethoxide, zirconium tetrabutoxide, bisindenylzirconium dichloride, triisopropoxyzirconium chloride, zirconium benzyl dichloride and tributoxyzirconium chloride, and hafnium compound includes tetrabenzyl hafnium, tetraethoxide hafnium and tetrabutoxide hafnium and further the vanadium compound includes bisacetylacetonatovanadium, triacetylacetonatovanadium, triethoxyvanadyl and tripropoxyvanadyl. Of these transition metal compounds, the titanium compounds are particularly preferred.

In the process of the present invention, if desired, in addition to the above transition metal compound, another catalytic component such as organic aluminum can be added.

The organic aluminum includes an organic aluminum compound represented by the formula:

R¹¹ ₖAl(OR¹)ₛHₚX_{q} (XI)

wherein R¹¹ and R¹ each independently represent an alkyl group having 1 to 8 carbon atoms, preferably 1 to 4 carbon atoms; X represents a halogen; k, s, p and q are 0<k≦3, 0≦s<3, 0≦p<3 and 0≦q<3, respectively, and k+s+p+q=3.

The activity of the catalyst is further improved by adding the above compound.

The organic aluminum compound represented by the above formula (XI) can be exemplified as shown below. Those corresponding to p=q=o are represented by the formula: R¹¹ₖAl(OR¹)₃₋ₖ (wherein R¹¹ and R¹ are the same as those mentioned above and k is preferably a number of 1.5≦k≦3). Those corresponding to s=p=o are represented by the formula: R¹¹ₖAlX₃₋ₖ (wherein R¹¹ and X are the same as those mentioned above and k is preferably a number of 0<k<3). Those corresponding to s=q=0 are represented by the formula: R¹¹ₖAlH₃₋ₖ (wherein R¹¹ is the same as mentioned above and k is preferably a number of 2≦k<3). Those corresponding to p=0 are represented by the formula: R¹¹ₖAl(OR¹)ₛXq (wherein R¹¹, R¹ and X are the same as those mentioned above and 0≦k<3, 0≦s<3, 0≦q<3 and k+s+q=3).

In the organic aluminum compound represented by the formula (XI), the compound wherein p=q=0 and k=3 is selected from, for example, trialkylaluminum such as triethylaluminum and tributylaluminum, or combination thereof, and those preferred are triethylaluminum, tri-n-butylaluminum and triisobutylaluminum. In the case of p=q=0 and 1.5≦k<3, included are dialkylaluminum alkoxide such as diethylaluminum ethoxide and dibutylaluminum butoxide; alkylaluminum sesquialkoxide such as ethylaluminum sesquiethoxide and butylaluminum sesquibutoxide; as well as partially alkoxylated alkylaluminum having an average composition represented by R¹ _{2.5} Al(OR¹³)_{0.5}. Examples of the compound corresponding to the case where s=p=0 include a partially halogenated alkylaluminum including dialkylaluminum halogenide (k=2) such as diethylaluminum chloride, dibutylaluminum chloride and diethylaluminum bromide; alkylaluminum sesquihalogenide (k=1.5) such as ethylaluminum sesquichloride, butylaluminum sesquichloride and ethylaluminum sesquibromide; and alkylaluminum dihalogenide (k=1) such as ethylaluminum dichloride, propylaluminum dichloride and butylaluminum dibromide. Examples of the oompound corresponding to the case in which s=q=0 includes a partially hydrogenated alkylaluminum including dialkylaluminum hydride (k=2) such as diethylaluminum hydride and dibutylaluminum hydride; alkylaluminum dihydride (s=k) such as ethylaluminum dihydride and propylaluminum dihydride. Examples of the compound corresponding to the case in which p=0 include a partially alkoxylated or halogenated alkylaluminum such as ethylaluminumethoxy chloride, butylaluminumbutoxy chloride and ethylaluminumethoxy bromide (k=s=q=1). Of these, triisobutylaluminum and diisobutylaluminum hydride are particularly suitable.

For practicing the process of the present invention, the amount of the alkylaluminoxane to be blended with the styrene monomer is not particularly limited, but preferably 0.001 to 1 mole per one liter of the styrene monomer. A ratio of alkylaluminoxane (the organic aluminum compound may be included) and the transition metal compound, in terms of the ratio of aluminum and titanium, i.e. aluminum/titanium (molar ratio), is 1 to 10⁶, and preferably 10 to 10⁴.

Polymerization (or copolymerization) of the styrene monomer may be carried out in bulk polymerization and may be carried out in the presence of a solvent, e.g. an aliphatic hydrocarbon such as pentane, hexane and heptane; an alicyclic hydrocarbon such as cyclohexane; and an aromatic hydrocarbon such as benzene, toluene and xylene. Moreover, the polymerization temperature is not particularly limited, but is usually 0 to 100°C, preferably 20 to 80°C. Polymerization (or copolymerization) of the styrene monomer is carried out by using only the styrene monomers which are purified by the purifying method of the present invention or by supplying other styrene monomers in the reaction system using the styrene monomers.

Further, in order to control the molecular weight of the resulting styrene polymer, it is effective to carry out the polymerization reaction in the presence of hydrogen.

The styrene based polymer thus obtained has a high degree of sydiotactic configuration. Specific examples of the styrene polymers include polystyrene, poly(alkylstyrene), poly(halogenated styrene), poly(alkoxystyrene), poly(vinyl benzoate) and mixtures thereof, and copolymers containing the same as main components. Here, the poly(alkylstyrene) includes poly(methylstyrene), poly(ethylstyrene), poly(isopropylstyrene) and poly(tert-butylstyrene), and the poly(halogenated styrene) includes poly(chlorostyrene), poly(bromostyrene) and poly(fluorostyrene). The poly(alkoxystyrene) includes poly(methoxystyrene) and poly(ethoxylstyrene). Of these, a particularly preferred styrene polymer includes polystyrene, poly(p-methylstyrene), poly(m-methylstyrene), poly(p-tert-butylstyrene), poly(p-chlorostyrene), poly(m-chlorostyrene), poly(p-fluorostyrene), and further a copolymer of styrene and p-methylstyrene.

The styrene polymer produced by the process of the present invention usually has a number average molecular weight of 1,000 to 5,000,000 preferably 50,000 to 4,000,000 and a high syndiotacticity as mentioned above. Furthermore, if necessary, after polymerization, when the resulting polymer is subjected to deashing treatment with a washing liquid containing hydrochloric acid and drying under reduced pressure to remove soluble components, a styrene polymer having a markedly large syndiotacticity and high purity can be obtained.

As mentioned above, according to the process of the present invention, since a styrene monomer containing less amount of impurities such as phenylacetylene compound is used as a starting material, catalyst activity can be markedly enhanced and high activity of the catalyst can be retained for a long period of term because the catalyst is not poisoned.

Therefore, when a styrene monomer is polymerized by using the purification method and polymerization method of the present invention, a styrene polymer having a high syndiotacticity can be prepared with good efficiency.

The styrene polymer having syndiotactic configuration thus obtained is excellent in various physical properties such as heat resistance and chemical resistance so that it can be utilized for various uses widely and effectively.

Next, the present invention will be described in more detail by referring to the following Examples.

### EXAMPLE 1

### (1) Purification of styrene

A crude styrene containing 140 ppm of phenylacetylene as an impurity was degassed under reduced pressure to decrease the water content to 2 ppm. It is also confirmed that the similar change was accomplished by passing an inert gas therethrough.

To 100 ml of the crude styrene subjected to the above pre-treatment was added 10 g of burned alumina, and the mixture was shaken at room temperature for 30 minutes. The content of phenylacetylene was confirmed to be lowered to 40 ppm by the gas chromatography analysis.

### (2) Polymerization of styrene

In a 30 ml of a vessel previously substituted with nitrogen was charged 10 ml of styrene monomer obtained by (1) above, and after elevating the temperature of the mixture to 70°C, 0.1 mmole of triisobutylaluminum and 0.1 mmole of methylaluminoxane were added to the mixture and then 0.527 µmole of pentamethylcyclopentadienyltitanium trimethoxide was added thereto to effect polymerization reaction at 70°C for 2 hours.

The polymerization reaction was stopped by adding methanol and the polymer was dried. Yield of the polymer (polystyrene) was 7.11 g and catalyst activity for polymerization was 297 kg/g.Ti.

### EXAMPLE 2

The same procedure was carried out as in (1) and (2) of Example 1 except for changing the processing time with activated alumina from 30 minutes to 60 minutes in (1) of Example 1.

Yield of the obtained polymer was 8.14 g and catalyst activity for polymerization was 340 kg/g.Ti.

### COMPARATIVE EXAMPLE 1

The same procedure was carried out as in (2) of Example 1 except for using crude styrene used in Example 1 without effecting the purification in (1) of Example 1.

Yield of the obtained polymer was 4.23 g and catalyst activity for polymerization was 177 kg/g.Ti.

### REFERENCE EXAMPLE 1

The same procedure was carried out as in (1) and (2) of Example 1 except for replacing activated alumina in (1) of Example 1 with zeolite 4A.

Yield of the obtained polymer was 5.01 g which is relatively good than the result of COMPARATIVE EXAMPLE 1 but the ratio of atactic configuration of the polymer raised to 18.7%.

### REFERENCE EXAMPLE 2

The same procedure was carried out as in (1) and (2) of Example 1 except for replacing activated alumina in (1) of Example 1 with zeolite 13X.

Yield of the obtained polymer was 5.42 g and the ratio of atacitc configuration of the polymer was 32.0%.

### EXAMPLE 3

### (1) Purification of styrene

A crude styrene containing 140 ppm of phenylacetylene as an impurity was charged in a closed vessel replaced by nitrogen and 0.5 mmole of methylaluminoxane was added per 100 ml of styrene, and the mixture was treated at 50°C for 30 minutes. Contact treatment was carried out by adding 30 g of burned alumina per 100 ml of the thus treated styrene to obtain purified styrene.

The content of phenylacetylene in the purified styrene was 8 ppm by the gas chromatography analysis.

### (2) Polymerization of styrene

In a closed vessel replaced by nitrogen was charged 10 ml of the purified styrene obtained in the above (1), and after elevating the temperature to 70°C, 75 µmole of triisobutylaluminum and 75 µmole of methylaluminoxane were added to the styrene, and then 0.375 µmole of pentamethyl-cyclopentadienyltitanium trimethoxide was added thereto and polymerization reaction was carried out at 70°C for one hour. Then, polymerization was stopped by adding methanol and the polymer was dried. Yield of the obtained polymer was 3.00 g and the catalyst activity was 138 kg/g Ti.

### EXAMPLE 4

### (1) Purificaton of styrene

A crude styrene containing 140 ppm of phenylacetylene as an impurity was charged in a closed vessel replaced by nitrogen and 1 mmole of triisobutylaluminum was added per 100 ml of styrene, and the mixture was treated at 50°C for one hour. Contact treatment was carried out by adding 30 g of burned alumina per 100 ml of the thus treated styrene to obtain purified styrene.

The content of phenylacetylene in the purified styrene was 5 ppm by the gas chromatography analysis.

### (2) Polymerization of styrene

In a closed vessel replaced by nitrogen was charged 10 ml of the purified styrene obtained in the above (1), and after elevating the temperature to 70°C, 75 µmole of triisobutylaluminum and 75 µmole of methylaluminoxane were added to the styrene, and then 0.375 µmole of pentamethyl-cyclopentadienyltitanium trimethoxide was added thereto and polymerization reaction was carried out at 70°C for one hour. Then, polymerization was stopped by adding methanol and the polymer was dried. Yield of the obtained polymer was 3.51 g and the catalyst activity was 161 kg/g Ti.

### EXAMPLE 5

### (1) Purification of styrene

Purification of styrene was carried out in the same manner as in (1) of EXAMPLE 4 except for using diisobutylaluminumhydride in place of triisobutylaluminum. The content of phenylacetylene in the purified styrene was not detectable by gas chromatography analysis.

### (2) Polymerization of styrene

Polymerization reaction was carried out in the same manner as in (2) of EXAMPLE 4 except for using purified styrene obtained in the above (1) of EXAMPLE. Yield of the obtained polymer was 3.94 g and the catalyst activity was 181 kg/g Ti.

## Claims

1. A process for producing styrene polymers having a high degree of syndiotactic configuration by polymerizing a styrene monomer in the presence of a catalyst composed of (A) aluminoxane and (B) a transition metal compound, selected from the group of zirconium, hafnium, titanium and vanadium compounds, characterized in that a purified styrene monomer containing a phenylacetylene compound in an amount of 50 ppm or less is used.

2. The process according to Claim 1, wherein said phenylacetylene compound is phenylacetylene.

3. The process according to Claim 1, wherein the amount of the phenylacetylene compound in the purified styrene monomer is 10 ppm or less.

4. The process according to Claim 1, wherein the purified styrene monomer is obtained by treating a crude styrene monomer for industrial use with at least one inorganic substance selected from the group consisting of alumina, activated alumina, silica gel, activated charcoal, calcium oxide, aluminum silicate and diatomaceous earth.

5. The process according to Claim 1, 2 or 3, wherein the purified styrene monomer is obtained by degassing a crude styrene monomer for industrial use under reduced pressure or passing a dried inert gas through the crude styrene by bubbling, and then treating it with at least one inorganic substance selected from the group consisting of alumina, activated alumina, silica gel, activated charcoal, calcium oxide, aluminum silicate and diatomaceous earth.

6. The process according to Claim 1, 2 or 3 wherein the purified styrene monomer is obtained by bringing a crude styrene monomer for industrial use into contact with an organoaluminum compound, and then treating it with at least one inorganic substance selected form the group consisting of alumina, activated alumina, silica gel, activated charcoal, calcium oxide, aluminum silicate and diatomaceous earth.

7. The process according to Claim 1, 2 or 3, wherein the purified styrene is obtained by bringing a crude styrene monomer for industrial use into contact with an organoaluminum compound and then distilling it.

8. The process according to Claim 1, 2 or 3, wherein the purified styrene is obtained by bringing a crude styrene monomer for industrial use into contact with an organoaluminum compound, hydrolyzing and then distilling it.

9. The process according to Claim 1, 2 or 3, wherein the purified styrene is obtained by bringing a crude styrene monomer for industrial use into contact with an organoaluminum compound, hydrolyzing and then treating it with at least one inorganic substance selected from the group consisting of alumina, activated alumina, silica gel, activated charcoal, calcium oxide, aluminum silicate and diatomaceous earth.

## Patentansprüche

1. Verfahren zur Herstellung von styrenpolymeren mit hohem Grad an syndiotaktischer Konfiguration durch Polymerisation eines Styrenmonomeren in Gegenwart eines Katalysators, bestehend aus (A) Aluminoxan (B) einer Übergangsmetallverbindung, ausgewählt aus der Gruppe, die aus Zirkon-, Hafnium-, Titan-und Vanadiumverbindungen besteht, dadurch gekennzeichnet, daß ein gereinigtes Styrenmonomeres verwendet wird, das eine Phenylacetylenverbindung in einer Menge von 50 ppm oder weniger enthält.

2. Verfahren nach Anspruch 1, worin die Phenylacetylenverbindung Phenylacetylen ist.

3. Verfahren nach Anspruch 1, worin die Menge der Phenylacetylenverbindung in dem gereinigten Styrenmonomeren 10 ppm oder weniger beträgt.

4. Verfahren nach Anspruch 1, worin das gereinigte Styrenmonomere erhalten wird durch Behandlung eines rohen Styrenmonomeren für industrielle Verwendung mit wenigstens einer anorganischen Substanz, ausgewählt aus der Gruppe, die aus Aluminiumoxid, aktiviertem Aluminiumoxid, Silicagel, Aktivkohle, Calciumoxid, Aluminiumsilicat und Diatomeen-Erde besteht.

5. Verfahren nach Anspruch 1, 2 oder 3, worin das gereinigte Styrenmonomere erhalten wird durch Entlüftung eines rohen Styrenmonomeren für industrielle Verwendung unter vermindertem Druck oder durch Hindurchperlen von trockenem Inertgas durch das rohe Styren, und anschließend Behandlung desselben mit wenigstens einer anorganischen Substanz, ausgewählt aus der Gruppe, die aus Aluminiumoxid, aktiviertem Aluminiumoxid, Silicagel, Aktivkohle, Calciumoxid, Aluminiumsilicat und Diatomeen-Erde besteht.

6. Verfahren nach Anspruch 1, 2 oder 3, worin das gereinigte Styrenmonomere erhalten wird, indem ein rohes Styrenmonomeres für die industrielle Verwendung in Kontakt gebracht wird mit einer Organoaluminiumverbindung, und dies anschließend mit wenigstens einer anorganischen Substanz behandelt wird, ausgewählt aus der Gruppe, die aus Aluminiumoxid, aktiviertem Aluminiumoxid, Silicagel, Aktivkohle, Calciumoxid, Aluminiumsilicat und Diatomeen-Erde besteht.

7. Verfahren nach Anspruch 1, 2 oder 3, worin das gereinigte Styren erhalten wird, indem ein rohes Styrenmonomeres für die industrielle Verwendung mit einer Organoaluminiumverbindung in Kontakt gebracht wird, und anschließend destilliert wird.

8. Verfahren nach Anspruch 1, 2 oder 3, worin das gereinigte Styren erhalten wird, indem ein rohes Styrenmonomeres für die industrielle Verwendung mit einer Organoaluminiumverbindung in Kontakt gebracht wird, hydrolysiert und anschließend destilliert wird.

9. Verfahren nach Anspruch 1, 2 oder 3, worin das gereinigte Styren erhalten wird, indem ein rohes Styrenmonomeres für die industrielle Verwendung wird mit einer Organoaluminiumverbindung in Kontakt gebracht wird, hydrolysiert wird und anschließend mit wenigstens einer anorganischen Substanz behandelt wird, ausgewählt aus der Gruppe, die aus Aluminiumoxid, aktiviertem Aluminiumoxid, Silicagel, Aktivkohle, Calciumoxid, Aluminiumsilicat und Diatomeen-Erde besteht.

## Revendications

1. Procédé pour la préparation de polymères de styrène ayant un taux élevé de configuration syndiotactique par polymérisation d'un monomère de styrène en présence d'un catalyseur composé (A) d'un aluminoxane et (B) d'un composé d'un métal de transition, choisi dans le groupe des composés du zirconium, du hafnium, du titane et du vanadium, caractérisé en ce qu'on utilise un monomère de styrène purifié contenant un composé de phénylacétylène en une quantité de 50 ppm ou moins.

2. Procédé selon la revendication 1, dans lequel ledit composé de phénylacétylène est le phénylacétylène.

3. Procédé selon la revendication 1, dans lequel la quantité de composé de phénylacétylène dans le monomère de styrène purifié est de 10 ppm ou moins.

4. Procédé selon la revendication 1, dans lequel le monomère de styrène purifié est obtenu en traitant un monomère de styrène brut pour usage industriel avec au moins une substance inorganique choisie dans le groupe constitué de l'alumine, de l'alumine activée, du gel de silice, du charbon actif, de l'oxyde de calcium, du silicate d'aluminium et de la terre de diatomées.

5. Procédé selon la revendication 1, 2 ou 3, dans lequel le monomère de styrène purifié est obtenu en dégazant un monomère de styrène brut pour usage industriel sous pression réduite ou en faisant passer un gaz inerte sec à travers le styrène brut par barbotage, et en le traitant ensuite avec au moins une substance inorganique choisie dans le groupe constitué par l'alumine, l'alumine activée, le gel de silice, le charbon actif, l'oxyde de calcium, le silicate d'aluminium et la terre de diatomées.

6. Procédé selon la revendication 1, 2 ou 3, dans lequel le monomère de styrène purifié est obtenu en amenant un monomère de styrène brut pour usage industriel en contact avec un composé d'organoaluminium, et en le traitant ensuite avec au moins une substance inorganique choisie dans le groupe constitué par l'alumine, l'alumine activée, le gel de silice, le charbon actif, l'oxyde de calcium, le silicate d'aluminium et la terre de diatomées.

7. Procédé selon la revendication 1, 2 ou 3, dans lequel le styrène purifié est obtenu en amenant un monomère de styrène brut pour usage industriel en contact avec un composé d'organoaluminium et ensuite en le distillant.

8. Procédé selon la revendication 1, 2 ou 3, dans lequel le styrène purifié est obtenu en amenant un monomère de styrène brut pour usage industriel en contact avec un composé d'organoaluminium, en l'hydrolysant et ensuite en le distillant.

9. Procédé selon la revendication 1, 2 ou 3,. dans lequel styrène purifié est obtenu en amenant un monomère de styrène brut pour usage industriel en contact avec un composé d'organoaluminium, en l'hydrolysant et en le traitant ensuite avec au moins une substance inorganique choisie dans le groupe constitué par l'alumine, l'alumine activée, le gel de silice, le charbon actif, l'oxyde de calcium, le silicate d'aluminium et la terre de diatomées.
